# EUROPÄISCHE PATENTSCHRIFT

(11) **EP 1 267 916 B1**
(45) Veröffentlichungstag und Bekanntmachung des Hinweises auf die Patenterteilung: **30.06.2004**
(21) Anmeldenummer: 01925576.9
(22) Anmeldetag: 04.04.2001
(51) Int. Cl.: A61K 38/48, A61K 38/17, A61P 19/00

(54) **ARZNEIMITTEL ENTHALTEND TISSUE INHIBITOR OF METALLOPROTEINASES-2 (TIMP-2) ALS OSTEOANABOL WIRKSAME SUBSTANZ**
MEDICAMENT CONTAINING A TISSUE INHIBITOR OF METALLOPROTEINASES-2 (TIMP-2) AS AN OSTEO-ANABOLICALLY ACTIVE SUBSTANCE
MEDICAMENT CONTENANT UN INHIBITEUR TISSULAIRE DES METALLOPROTEINASES-2 (TIMP-2) EN TANT QUE SUBSTANCE OSTEO- ANABOLISANTE

(30) Priorität: 05.04.2000 DE 10016791
(43) Veröffentlichungstag der Anmeldung: 02.01.2003
(73) Patentinhaber: IPF Pharmaceuticals GmbH, 30625 Hannover (DE)
(72) Erfinder: KRAMER, Franz-Josef, 31832 Springe (DE); MARK, Silke, 34587 Felsberg-Gensungen (DE); STÄNDKER, Ludger, 30635 Hannover (DE); FORSSMANN, Wolf-Georg, 30175 Hannover (DE)
(74) Vertreter: Meyers, Hans-Wilhelm, Dr.Dipl.-Chem.
(86) Internationale Anmeldenummer: PCT/EP2001/004891
(87) Internationale Veröffentlichungsnummer: WO 2001/074380

(56) Entgegenhaltungen:
- WO-A-98/04287
- US-A- 5 595 885
- US-A- 5 643 752
- US-A- 5 714 465
- US-A- 5 766 585

## Beschreibung

Die Erfindung betrifft ein Arzneimittel und ein Diagnostikmittel enthaltend Tissue Inhibitor of Metalloproteinases-2 (TIMP-2) als osteoanabol wirksames Peptid-Präparat und Verwendungen desselben.

TIMP-2 gehört zur Familie der "Tissue inhibitors of metalloproteinases", von welcher bisher vier verschiedene Peptide (TIMP-1 bis -4) mit unterschiedlichen biologischen Funktionen bekannt sind.

Die Primärstruktur von TIMP-2 konnte in verschiedenen Organismen wie Mensch (Liotta et al., 1991), Ratte (Roswit et al., 1992) und Maus (Kishi et al., 1991) aufgeklärt werden. Das Peptid enthält insgesamt 12 Cysteine, die über 6 Cysteinbrücken miteinander verknüpft sind.

Zu den biologischen Funktionen von TIMP-2 gehört vor allem die Inhibition von aktiven Matrix-Metalloproteinasen (MMPs). Unter MMPs versteht man eine Gruppe von Zink-abhängigen Endoproteinasen, die in der Lage sind, die Extrazellularmatrix abzubauen. Die Extrazellularmatrix besteht aus einer komplexen Struktur und wird unter anderem aus Collagen, Proteoglycanen, Glycoproteinen und Glycosaminoglycanen gebildet. Ein Abbau der Extrazellularmatix ist essentiell für viele biologische Prozesse wie Embryogenese, Morphogenese sowie Geweberesorption und -remodellierung. Allerdings kann eine unregulierte Aktivität der MMPs zu einer beträchtlichen Zerstörung von Geweben führen wie beispielsweise bei rheumatischer Arthritis (Okada et al., 1986).

Mit der Inhibition der Matrix-Metalloproteinasen stehen weitere biologische Funktionen von TIMP-2 im Zusammenhang, wozu unter anderem die Reduktion des Wachstums von Tumorzellen (Gomez et al., 1997) sowie die Inhibition der Angiogenese (Valente et al., 1998) zählen.

Neben diesen Funktionen sind auch intrinsische, biologische Aktivitäten von TIMP-2 beschrieben. Dazu zählen eine vermehrte Bildung von roten Blutkörperchen (Stetler-Stevenson et al., 1992) sowie eine mitogene Wirkung auf verschiedene Zelllinien (Hayakawa et al., 1994). Weiterhin konnte in vitro sowohl eine Inhibition der Knochenresorption in Gesamtknochenkulturen (Hill et al., 1993), als auch eine Stimulation der Knochenresorption durch Osteoklasten (Shibutani et al., 1999) gezeigt werden.

Die US-A-5,714,465 betrifft die Behandlung von Knochenerkrankungen mit pharmazeutischen Zusammensetzungen, die TIMP-2 enthalten.

Auch die US-A-5,595,885 betrifft pharmazeutische Zusammensetzungen, die TIMP-2 enthalten.

Die US-A-5,643,752 betrifft die Verwendung von Matrix-Metalloproteinasen, insbesondere TIMP-4, zur Behandlung verschiedener Knochenerkrankungen.
Fig. 1: A) bis D) Die unterschiedlichen Chromatographieschritte zur Aufreinigung des osteoproliferativen TIMP-2.
Fig. 2: A) Proliferative Wirkung von rekombinantem humanem TIMP-2 auf primäre, fetale Rattenosteoblasten. B) Einfluss von rekombinantem humanem TIMP-2 auf den mittleren Zelldurchmesser bei der osteoblastären Zellinie der Maus MC3T3-E1.

Überraschenderweise ist TIMP-2 mit der Aminosäuresequenz 
Z¹CSCSPVHPQQAFCNADVVIRAKAVSEKEVDSGNDIYGNPIKRIQYEIKQIKMFKGP
X1KDIEFIYTAPSSAVCGVSLDVGGKKEYLIAGKAEGDGKMHITLCDFIVPWDTLX2TT
QKKSLNHRYQMGCECKITRCPMIPCYISSPDECLWMDWVTEKX3INGHQAKFFACIKR
SDGSCAWYRGAAPP KQEFLDIEDP-Z²
 wobei
- X1: die Aminosäure E oder D
- X2: die Aminosäure T oder I
- X3: die Aminosäure N oder S
- Z¹: -NH₂, substituiertes Amin oder ein beliebiges Peptid mit bis zu zehn Aminosäuren,
- Z²: -COOH, CONH₂, substituiertes Amid oder ein beliebiges Peptid mit bis zu zehn Aminosäuren bedeutet,
sowie von dessen biologisch aktiven Fragmenten und/oder amidierten, acylierten, sulfatierten, phosphorylierten, glykosylierten und/oder Polyethylenglykol-modifizierten Derivaten, in der Lage eine Stimulation der für den Knochenaufbau verantwortlichen Osteoblasten nach Knochenfrakturen oder operativen Eingriffen zu bewirken. TIMP-2 wirkt dabei proliferativ und protektiv auf fetale Osteoblasten.

Erfindungsgemäß verwendet wird ein Arzneimittel enthaltend TIMP-2 mit der Aminosäuresequenz 
Z¹CSCSPVHPQQAFCNADVVIRAKAVSEKEVDSGNDIYGNPIKRIQYEIKQIKMFKGP
X1KDIEFIYTAPSSAVCGVSLDVGGKKEYLIAGKAEGDGKMHITLCDFIVPWDTLX2TT
QKKSLNHRYQMGCECKITRCPMIPCYISSPDECLWMDWVTEKX3INGHQAKFFACIKR
SDGSCAWYRGAAPP KQEFLDIEDP-Z²
 wobei
- X1: die Aminosäure E oder D
- X2: die Aminosäure T oder I
- X3: die Aminosäure N oder S
- Z¹: -NH₂, substituiertes Amin oder ein beliebiges Peptid mit bis zu zehn Aminosäuren,
- Z²: -COOH, CONH₂, substituiertes Amid oder ein beliebiges Peptid mit bis zu zehn Aminosäuren bedeutet,
sowie von dessen biologisch aktiven Fragmenten und/oder amidierten, acylierten, sulfatierten, phosphorylierten, glykosylierten und/oder Polyethylenglykol-modifizierten Derivaten.

Bevorzugt verwendet werden erfindungsgemäß humanes TMP-2 (SEQ. ID. No 1) und TIMP-2 der Ratte (SEQ. ID. No 2) :

CSCSPVHPQQAFCNADVVIRAKAVSEKEVDSGNDIYGNPIKRIQYEIKQIKMFKGPEKD
IEFIYTAPSSAVCGVSLDVGGKKEYLIAGKAEGDGKMHITLCDFIVPWDTLSTTQKKSLN
HRYQMGCECKITRCPMIPCYISSPDECLWMDWVTEKNINGHQAKFFACIKRSDGSCA
WYRGAAPPKQEFLDIEDP (SEQ. ID. NO 1)

CSCSPVHPQQAFCNADVVIRAKAVSEKEVDSGNDIYGNPIKRIQYEIKQIKMFKGPDKD
IEFIYTAPSSAVCGVSLDVGGKKEYLIAGKAEGDGKMHITLCDFIVPWDTLSITQKKSLN
HRYQMGCECKITRCPMIPCYISSPDECLWMDWVTEKSINGHQAKFFACIKRSDGSCA
WYRGAAPPKQEFLDIEDP (SEQ. ID. NO 2)

Gegenstand der Erfindung ist die Verwendung von TIMP-2 mit der Aminosäuresequenz 
Z¹CSCSPVHPQQAFCNADVVIRAKAVSEKEVDSGNDIYGNPIKRIQYEIKQIKMFKGP
X1KDIEFIYTAPSSAVCGVSLDVGGKKEYLIAGKAEGDGKMHITLCDFIVPWDTLX2TT
QKKSLNHRYQMGCECKITRCPMIPCYISSPDECLWMDWVTEKX3INGHQAKFFACIKR
SDGSCAWYRGAAPP KQEFLDIEDP-Z²
 wobei
- X1: die Aminosäure E oder D
- X2: die Aminosäure T oder I
- X3: die Aminosäure N oder S
- Z¹: -NH₂, substituiertes Amin oder ein beliebiges Peptid mit bis zu zehn Aminosäuren,
- Z²: -COOH, CONH₂, substituiertes Amid oder ein beliebiges Peptid mit bis zu zehn Aminosäuren bedeutet,
sowie von dessen biologisch aktiven Fragmenten und/oder amidierten, acylierten, sulfatierten, phosphorylierten, glykosylierten und/oder Polyethylenglykol-modifizierten Derivaten zur Herstellung eines Arzneimittels zur Stimulation von Osteoblasten nach Knochenfrakturen oder operativen Eingriffen.

Erfindungsgemäß verwendbare TIMP-2 Derivate weisen vorzugsweise mindestens 90% Sequenzidentität zu der nativen Sequenz des TIMP-2 oder der genannten Modifizierungen auf.

Es kann vorteilhaft sein das erfindungsgemäß zu verwendende TIMP-2 gemäß in Kombination mit anderen Wachstumsfaktoren und / oder Arzneimitteln sowie in Kombination mit medizinischen Hilfsmitteln, beispielsweise bei Implantaten einzusetzen.

Die Verabreichung des TIMP-2 erfolgt insbesondere als Zubereitung in Form von Injektionen, Salben, "slow release"-Kapseln und ähnlichen galenischen Formulierungen.

Das erfindungsgemäß zu verwendende TIMP-2 wird zur Herstellung eines osteoanabolen Arzneimittels zur Behandlung von Knochendefekten und zur Verbesserung der Knochenregeneration nach Knochenfrakturen oder operativen Eingriffen, eingesetzt.

Die Grundlage für die Identifizierung von TIMP-2 als osteoanabolem Peptid bildete die Beobachtung, dass eine unterschiedliche Regenerationsfähigkeit von Knochendefekten in Abhängigkeit des Alters eines Organismus besteht. Während adulte Organismen normalerweise nicht in der Lage sind, größere Knochendefekte durch die Bildung von neuem Knochengewebe zu regenerieren, findet bei jüngeren Organismen eine vollständige, knöcherne Regeneration von vergleichbaren Defekten statt.

Ähnliche Beobachtungen wurden auch in vitro bei der Untersuchung von Osteoblasten erhalten: Aus fetalen Organismen isolierte Osteoblasten wachsen in Kultur problemlos und beginnen nach wenigen Tagen zu mineralisieren. Osteoblasten aus immaturen und adulten Tieren sind hingegen unter Standard-Kulturbedingungen nicht in der Lage zu proliferieren und sterben schließlich. Supplementiert man diese allein nicht lebensfähigen Zellen mit dem Überstand von fetalen Osteoblasten, so proliferieren und mineralisieren diese ähnlich wie die fetalen Zellen.

Ein deutlich regenerative Fähigkeit von dem Zellkulturüberstand fetaler Osteoblasten konnte auch in vivo gezeigt werden. Dabei wurde Ratten operativ ein Schädeldefekt zugefügt, der ohne äußere Stimulation nicht unter Bildung von Knochengewebe ausheilen kann. Appliziert man jedoch in diese Defekte einen Peptidextrakt aus dem Zellkulturüberstand von fetalen, primären Osteoblasten, so findet eine vollständige Regeneration des Defekts unter Ausbildung von neuem Knochengewebe statt.

Um die Substanz zu isolieren, die für die oben beschriebenen Effekte verantwortlich ist, wurde eine Peptidextraktion aus dem Überstand von fetalen Osteoblasten durchgeführt und die erhaltenen Peptide anschließend in vitro auf ihre osteoproliferativen Fähigkeiten hin untersucht (siehe Beispiel 3). Dabei konnte das erfindungsgemäße Peptid mittels präparativer, semipräparativer und analytischer RP-Chromatographie anhand seiner biologischen Aktivität aufgereinigt werden.

Die biochemische Charakterisierung des aufgereinigten, erfindungsgemäßen Peptids erfolgte durch Massenspektrometrie und N-terminale Sequenzierung. Die Bestimmung der Molekularmasse mittels Elektrospray-Massenspektrometrie ergab ein Molekulargewicht von 21715 Da. Die N-terminale Sequenzanalyse mittels Edman-Abbau resultierte in folgender Sequenz:

XSXSPVHPQQAFXNADVVIRAKAV

Der erfindungsgemäße Stoff kann zu einem verstärkten Wachstum und zu einer verbesserten Regeneration von Knochengewebe eingesetzt werden. Dies ist nach Knochenfrakturen oder operativen Eingriffen denkbar.

Die Erfindung wird anhand der folgenden Beispiele näher erläutert:

### Beispiel 1: Isolierung von TIMP-2 aus Zellkulturüberstand

Zellkulturüberstände wurden von konfluenten Kulturen primärer fetaler Osteoblasten gewonnen. Dazu wurden die Osteoblasten zunächst aus den Schädeldecken von Rattenfeten durch sequentiellen Verdau mit Collagenase und Trypsin isoliert und anschließend in Minimal Essential Medium Eagle (MEM) mit Penicillin/Streptomycin und 10% fetalem Kälberserum (FCS) kultiviert. Nach Erreichen der Konfuenz wurden die Zellen im Wechsel jeweils 24 h mit 10% FCS und 24 h serumfrei gehalten. Die serumfreien Überstände wurden dabei gesammelt und zur weiteren Peptidisolierung verwendet.

Die Isolierung von TIMP-2 erfolgte durch verschiedene aufeinanderfolgende RP-HPLC-Schritte, die zur Fraktionierung des Gesamtpeptidextraktes dienten. Aliquots der Fraktionen wurden im Bioassay getestet. Die Fraktionen wurden bei -20°C gelagert.

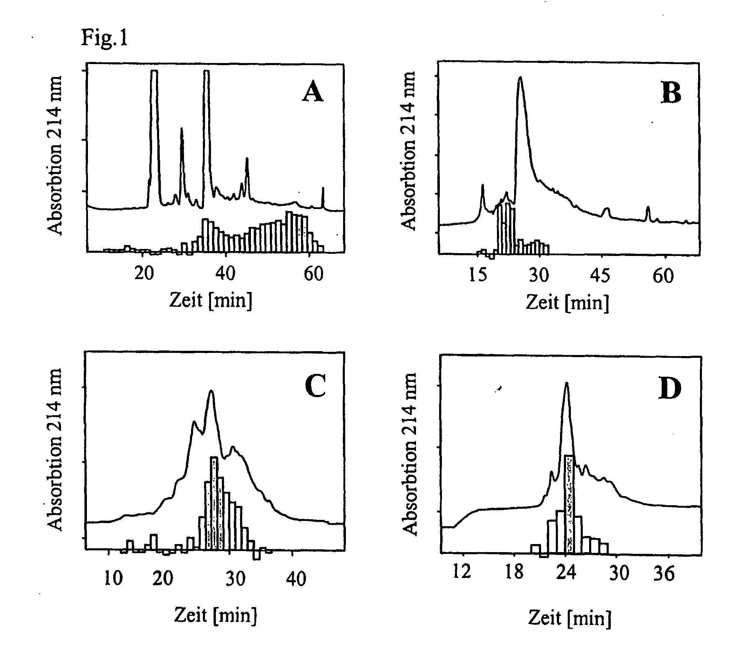

Die unterschiedlichen Chromatographieschritte zur Aufreinigung des osteoproliferativen TIMP-2 sind in der Fig. 1 A-D gezeigt.

### 1. Präparative Chromatographie

Die serumfreien Zellkulturüberstände wurden mit HCl auf einen pH von 2,5 und durch Zugabe von Acetonitril auf 5% Acetonitril eingestellt. Daraufhin wurden sie zunächst über einen Glasfaserfilter (GF6, Schleicher&Schuell) und anschließend über einen Membranfilter (OE G7, 4,43 µm, Schleicher&Schuell) filtriert. Das so erhaltene Material wurde in Mengen von 1-2 l mit einer Flussrate von 40 bis 50 ml/min auf eine RP-Säule aufgetragen.

| Chromatographiebedingungen: | |
|---|---|
| Säule | YMC Gel Basic (15 - 30 µm, 47 x 300 mm) |
| Fluss | 40 ml/min |
| Fraktionen | 50 ml |
| Puffer A | 10 mM HCl |
| Puffer B | 80% (w/v) Acetonitril, 10 mM HCl |
| Gradient | 5 - 75 % B in 47,5 min |
| | 75 - 100 % B in 5 min |

Anschließend wurden Aliquots der erhaltenen Fraktionen im Bioassay getestet.

### 2. Semipräparative Auftrennung

Die im Assay bioaktiven Fraktionen 37-40 wurden mit Hilfe eines weiteren RP-Schrittes aufgetrennt.

| Chromatographiebedingungen: | |
|---|---|
| Säule | Biotek RP Silica C4 (100 A, 5 µm, 20 x 125 mm) |
| Fluss | 5 ml/min |
| Fraktion | 5 ml |
| Puffer A | 0,1% TFA |
| Puffer B | 80% (w/v) Acetonitril, 0,1% TFA |
| Gradient | 5 - 40 % B in 10 min |
| | 40 - 100 % B in 60 min |

Aliquots der erhaltenen Fraktionen wurden wiederum im Bioassay getestet.

### 3. Analytische Chromatographie

Die nachfolgende Fraktionierung der bioaktiven Fraktionen 22 und 23 erfolgte mit Hilfe einer weiteren RP-Chromatographie.

| Chromatographiebedingungen: | |
|---|---|
| Säule | YMC C18 (120 A, 5 µm, 4,6 x 250 mm) |
| Fluss | 0,6 ml/min |
| Fraktion | 0,6 ml |
| Puffer A | 0,1% TFA |
| Puffer B | 80% (w/v) Acetonitril, 0,1% TFA |
| Gradient | 5 - 30 % B in 4 min |
| | 30 - 55 % B in 50 min |
| | 55 - 100 % B in 5 min |

Aliquots der erhaltenen Fraktionen wurden im Bioassay getestet.

### 4. Analytische Chromatographie

Die bioaktiven Fraktionen 27-29 wurden erneut mittels RP-Chromatographie aufgetrennt.

| Chromatographiebedingungen: | |
|---|---|
| Säule | Phenomenex C5 (4,6 x 250 mm) |
| Fluss | 0,6 ml/min |
| Fraktion | 0,6 ml |
| Puffer A | 0,1% TFA |
| Puffer B | 80% (w/v) Acetonitril, 0,1% TFA |
| Gradient | 5 - 38 % B in 4 min |
| | 38 - 58 % B in 60 min |
| | 58 - 100 % B in 5 min |

Im anschließenden Biotest konnte das erfindungsgemäße Peptid in Fraktion 24 in reiner Form erhalten werden.

### Beispiel 2: Biochemische Analyse von TIMP-2

### 1. Massenbestimmung

Die Massenbestimmung des nativen, aufgereinigten Peptids wurden mit Hilfe eines ESI-Massenspektrometers durchgeführt. Es wurde eine Molekularmasse von 21715 Da erhalten. Diese stimmt sehr gut mit der theoretischen Masse der oxidierten Form von TIMP-2 überein, in der alle Cysteine über Cysteinbrücken verbrückt sind.

### 2. Sequenzbestimmung

Das aufgereinigte, native Peptid wurde mittels Edmann-Abbau auf einem ABI 494 Sequenzer unter Verwendung des Standard-Programms analysiert. Die ersten 24 Abbauschritte ergaben folgende N-terminale Sequenz:

XSXSPVHPQQAFXNADVVIRAKAV

Bei X handelt es sich dabei um Cysteinreste, die bei der Sequenzierung nicht detektierbar sind. Unter Berücksichtigung dieser nicht detektiebaren Cysteine, stimmt die erhaltene Sequenz exakt mit der bekannten Sequenz von TIMP-2 überein. Nebensequenzen wurden nicht erhalten.

### 3. Datenbankrecherche

Bei anschließendem Abgleich in der Datenbank SwissProt wurde die Sequenz eindeutig als Beginn von TIMP-2 identifiziert. Die theoretische Masse des Proteins von 21713 Da stimmt ebenfalls mit der gemessen Masse von 21715 Da überein. Das aufgereinigte Peptid konnte somit eindeutig als TIMP-2 identifiziert werden.

### Beispiel 3: Bestimmung der biologischen Aktivität von TIMP-2

Die Isolierung von TIMP-2 erfolgte anhand seiner biologischen Aktivität, wobei die Proliferation fetaler Rattenosteoblasten gemessen wurde.

Dazu wurden Aliquots der erhaltenen Peptidfraktionen lyophilisiert und in serumfreiem Medium resuspendiert. Anschließend wurde ihre proliferative Wirkung auf fetale Osteoblasten getestet. Fraktionen mit proliferativer Wirkung wurden daraufhin einer weiteren chromatographischen Auftrennung unterzogen. Die unterschiedlichen Chromatographieschritte zur Aufreinigung des osteoproliferativen TIMP-2 sind in der Fig. 1 A-D gezeigt.

Für die Proliferationsmessung wurden die primären Osteoblasten trypsiniert und in einer Zellzahl von 5000 Zellen pro Well in 96-Well-Platten in serumfreiem Medium ausplattiert. Im Anschluss wurden die zu testenden Fraktionen zugegeben. Nach einer Inkubationszeit von 48 h oder 72 h wurde die Proliferation mittels zweier verschiedener Methoden getestet. Als Positivkontrollen wurden verschiedenen Konzentrationen an fetalem Kälberserum und TGFβ1 verwendet. Als Negativkontrolle dienten Zellen ohne Stimulation.

Die Proliferationsmessung erfolgte zum einen mit Hilfe des Wst-1-Substrates. Dieses Substrat wird von mitochondrialen Enzymen in stoffwechselaktiven Zellen zu einem farbigen Produkt umgesetzt, wobei die entstehende Farbintensität der Zellzahl proportional ist. Die Farbintensität wird bei einer Wellenlänge von 405 nm und einer Referenzwellenlänge von über 600 nm im ELISA-Reader bestimmt.

Zum anderen wird die Proliferation über eine direkte Messung der Zellzahl in einem Coulter Counter (CASY) gemessen.

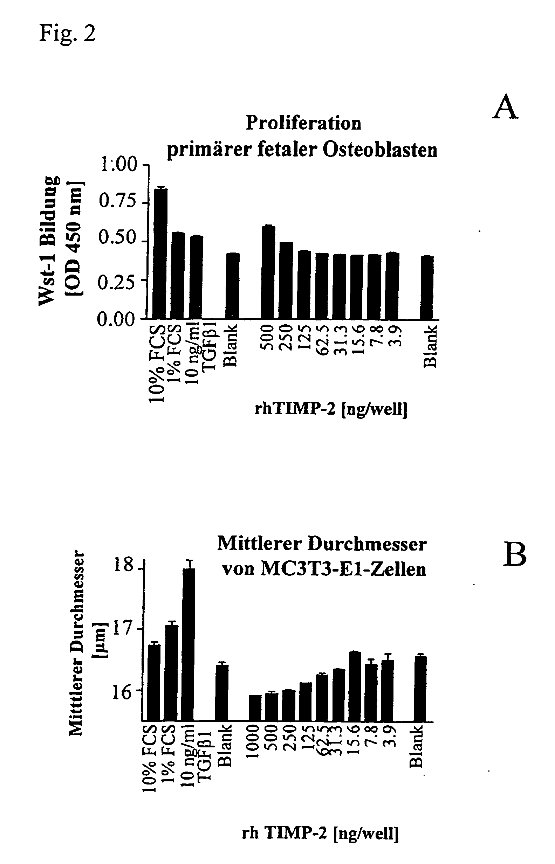

Beide Methoden zeigten eine dosisabhängige, wachstumsfördernde Wirkung von rekombinantem, humanen TIMP-2 auf Osteoblasten (Fig. 2A). Eine signifikante osteoproliferative Wirkung von TIMP-2 ist ab Konzentrationen von 100 ng/ml in diesen in vitro Experimenten nachzuweisen. Darüber hinaus bewirkt rekombinantes, humanes TIMP-2 eine Abnahme des mittleren Zelldurchmessers bei der osteoblastären Zellinie der Maus MC3T3-E1 (Fig. 2B). Signifikante Wirkungen vonTIMP-2 können ab Konzentrationen von 100 ng/ml in diesem in vitro Experiment nachzuweisen werden. Weiterhin konnte mikroskopisch eine protektive und proliferative Wirkung auf die osteoblastären Zelllinie der Maus MC3T3-E1 gezeigt werden.

Da alle diese Zellen typische Knochenzeilen sind, kann TIMP-2 als osteoanaboler Faktor angesehen werden.

## Patentansprüche

1. Verwendung von TIMP-2 mit der Aminosäuresequenz 
Z¹CSCSPVHPQQAFCNADVVIRAKAVSEKEVDSGNDIYGNPIKRIQYEIKQIKMFKGP
X1KDIEFIYTAPSSAVCGVSLDVGGKKEYLIAGKAEGDGKMHITLCDFIVPWDTLX2TT
QKKSLNHRYQMGCECKITRCPMIPCYISSPDECLWMDWVTEKX3INGHQAKFFACIKR
SDGSCAWYRGAAPP KQEFLDIEDP-Z²
 wobei
X1 die Aminosäure E oder D
X2 die Aminosäure T oder I
X3 die Aminosäure N oder S
Z¹ -NH₂, substituiertes Amin oder ein beliebiges Peptid mit bis zu zehn Aminosäuren,
Z² -COOH, CONH₂, substituiertes Amid oder ein beliebiges Peptid mit bis zu zehn Aminosäuren bedeutet,
sowie von dessen biologisch aktiven Fragmenten und/oder amidierten, acylierten, sulfatierten, phosphorylierten, glykosylierten und/oder Polyethylenglykol-modifizierten Derivaten zur Herstellung eines Arzneimittels zur Stimulation von Osteoblasten nach Knochenfrakturen oder operativen Ergriffen.

2. Verwendung nach Anspruch 1, wobei das TIMP-2 Derivat mindestens 90% Sequenzidentität zu der Sequenz gemäß Anspruch 1 besitzt.

3. Verwendung von TIMP-2 gemäß Anspruch 1 und/oder 2 in Kombination mit anderen Wachstumsfaktoren und / oder Arzneimitteln sowie in Kombination mit medizinischen Hilfsmitteln, beispielsweise bei Implantaten.

4. Verwendung von TIMP-2 gemäß Anspruch 1 bis 3 zubereitet für Injektionen, Salben, "slow release"-Kapseln und ähnlichen galenischen Zubereitungen.

## Claims

1. Use of TIMP-2 having the amino acid sequence 
Z¹CSCSPVHPQQAFCNADVVIRAKAVSEKEVDSGNDIYGNPIKRIQYEIKQIKMFKGP
X1KDIEFIYTAPSSAVCGVSLDVGGKKEYLIAGKAEGDGKMHITLCDFIVPWDTLX2TT
QKKSLNHRYQMGCECKITRCPMIPCYISSPDECLWMDWVTEKX3INGHQAKFFACIKR
SDGSCAWYRGAAPP KQEFLDIEDP-Z²
 wherein
X1 represents the amino acid E or D;
X2 represents the amino acid T or I;
X3 represents the amino acid N or S;
Z¹ represents -NH₂, a substituted amine or an arbitrary peptide having up to ten amino acids;
Z² represents -COOH, CONH₂, a substituted amide or an arbitrary peptide having up to ten amino acids;
and its biologically active fragments and/or amidated, acylated, sulfated, phosphorylated, glycosylated and/or polyethylene-glycol modified derivatives for preparing a medicament for stimulating osteoblasts after bone fractures or surgical intervention.

2. The use according to claim 1, wherein said TIMP-2 derivative has at least 90% sequence identity with the sequence according to claim 1.

3. The use of TIMP-2 according to claims 1 and/or 2 in combination with other growth factors and/or medicaments and in combination with medical aids, for example, in grafts.

4. The use of TIMP-2 according to claims 1 to 3 formulated for injections, ointments, sustained release capsules and similar galenic formulations.

## Revendications

1. Utilisation du TIMP-2 avec la séquence d'acides aminés 
Z¹CSCSPVHPQQAFCNADVVIRAKAVSEKEVDSGNDIYGNPIKRIQYEIKQIKMFKGP
X1KDIEFIYTAPSSAVCGVSLDVGGKKEYLIAGKAEGDGKMHITLCDFIVPWDTLX2TT
QKKSLNHRYQMGCECKITRCPMIPCYISSPDECLWMDWVTEKX3INGHQAKFFACIK
RSDGSCAWYRGAAPP KQEFLDIEDP-Z²
X1 désignant l'acide aminé E ou D
X2, désignant l'acide aminé T ou I,
X3, désignant l'acide aminé N ou S,
Z¹, désignant -NH₂, une amine substituée ou un peptide quelconque ayant jusqu'à dix acides aminés,
Z², désignant -COOH, CONH₂, un amide substitué ou un peptide quelconque ayant jusqu'à dix acides aminés,
ainsi que de ses fragments biologiquement actifs et/ou dérivés amidés, acylés, sulfatés, phosphorylés, glycosylés et/ou modifiés avec du polyéthylèneglycol pour la fabrication d'un médicament destiné à la stimulation d'ostéoblastes suite à des fractures osseuses ou à des interventions chirurgicales.

2. Utilisation selon la revendication 1, le dérivé du TIMP-2 possédant au moins 90 % d'identité de séquence avec la séquence selon la revendication 1.

3. Utilisation du TIMP-2 selon la revendication 1 et/ou 2 combiné à d'autres facteurs de croissance et/ou médicaments ainsi que combiné à des moyens auxiliaires médicaux, par exemple, dans des implants.

4. Utilisation du TIMP-2 selon les revendications 1 à 3, préparé pour être administré sous forme d'injections, de pommades, de gélules « à libération lente » et de préparations galéniques analogues.
